# EUROPÄISCHE PATENTSCHRIFT

(11) **EP 4 077 213 B1**
(45) Veröffentlichungstag und Bekanntmachung des Hinweises auf die Patenterteilung: **18.06.2025**
(21) Anmeldenummer: 20829892.7
(22) Anmeldetag: 16.12.2020
(51) Int. Cl.: C01B 17/88, C01B 17/90, C01B 21/44, C01B 21/46, F25B 47/00, F28F 19/02, F28G 9/00, F28D 21/00

(54) **VERFAHREN ZUR REINIGUNG EINER IN DER AUFKONZENTRIERUNG EINER MINERALSÄURE EINGESETZTEN VORRICHTUNG**
METHOD FOR PURIFYING A DEVICE USED IN THE CONCENTRATION OF A MINERAL ACID
PROCÉDÉ DE NETTOYAGE D'UN DISPOSITIF UTILISÉ LORS DE LA CONCENTRATION D'UN ACIDE MINÉRAL

(30) Priorität: 18.12.2019 EP 19217596
(43) Veröffentlichungstag der Anmeldung: 26.10.2022
(73) Patentinhaber: Covestro Deutschland AG, 51373 Leverkusen (DE)
(72) Erfinder: PENNEMANN, Bernd, 51467 Bergisch Gladbach (DE); TAJVIDI, Kameh, 41468 Neuss (DE)
(74) Vertreter: Levpat
(86) Internationale Anmeldenummer: PCT/EP2020/086459
(87) Internationale Veröffentlichungsnummer: WO 2021/122773

(56) Entgegenhaltungen:
- WO-A1-2011/032659
- JP-A- 2018 151 132
- KR-A- 20190 004 557

## Beschreibung

Die vorliegende Erfindung betrifft ein Verfahren zur Vorbereitung einer Vorrichtung (oder einer Anlage umfassend mehrere Vorrichtungen), beispielsweise eines Wärmeaustauschers, eines Verdampfers oder einer Destillationskolonne, für deren Einsatz in einem (insbesondere kontinuierlich betriebenen) Prozess zur Aufkonzentrierung einer Mineralsäure durch Verdampfen von Wasser, wobei die Vorrichtung bzw. die Anlage (mindestens) eine gegen die Mineralsäure beständige Einrichtung umfasst und die Einrichtung mit einer wässrigen Alkalimetallhydroxidlösung einer Massenkonzentration an Alkalimetallhydroxid im Bereich von 1 % bis 30 % bei einer Temperatur im Bereich von 40 °C bis 90 °C für einen Zeitraum von 2 Stunden bis 7 Tagen gespült wird.

Vorrichtungen, die in der Aufkonzentrierung einer Mineralsäure eingesetzt werden (zum Beispiel Wärmeaustauscher, Verdampfer oder Destillationskolonnen), werden in Prozessen benötigt, in denen eine Mineralsäure Anwendung findet und die Mineralsäure im Zuge dieser Anwendung verdünnt, jedoch nicht (zumindest nicht vollständig) verbraucht wird. Um eine Wiederverwendung der Mineralsäure zu ermöglichen, muss diese wieder auf die ursprüngliche Konzentration aufkonzentriert (und gegebenenfalls gereinigt) werden. So findet beispielsweise Schwefelsäure Anwendung in verschiedenen Einsatzgebieten, bei denen diese nicht chemisch verbraucht, aber mit Wasser verdünnt wird, etwa bei Einsatz als Trockenmittel oder als Katalysator in Reaktionen, bei denen Wasser gebildet wird. Bestimmte Bestandteile der zur Aufkonzentrierung der Mineralsäure eingesetzten Vorrichtungen kommen während des Betriebs derselben zwangsläufig mit der Mineralsäure direkt in Kontakt. Daher umfassen derartige Vorrichtungen stets auch Einrichtungen, die aus säurebeständigen Materialien gefertigt oder zumindest mit solchen beschichtet sind. Erfahrungsgemäß müssen insbesondere die säurebeständigen Einrichtungen derartiger Vorrichtungen regelmäßig gereinigt werden, insbesondere um Ablagerungen von Feststoffen zu entfernen.

Hierzu ist es üblich, die Vorrichtung oder sogar die säurebeständigen Einrichtungen selbst zumindest teilweise auseinanderzunehmen und wo erforderlich mit Wasser unter Hochdruck zu reinigen. Da jedoch die erforderlichen säurebeständigen Materialien durchaus eine gewisse Empfindlichkeit gegenüber mechanischen Belastungen aufweisen, kann dies zu Beschädigungen führen. Darüber hinaus ist ein Reinigungsverfahren, das ein mechanisches Auseinandernehmen auch nur einzelner Bauteile der Vorrichtung erfordert, natürlich umständlich und mit hohem Zeitaufwand verbunden.

Eine Reinigung ohne ein Auseinandernehmen mindestens einzelner Bauteile und ohne mechanische Belastungen ist mit Wasser allein kaum möglich. Dem Einsatz von Reinigungschemikalien sind jedoch durch die Beständigkeit der eingesetzten Werkstoffe Grenzen gesetzt.

CN 104745337 A beschreibt ein neutrales chemisches Reinigungsmittel zum Beseitigen von harten Ablagerungen eines Luftvorwärmers eines Entschwefelungssystems und ein Herstellungsverfahren des neutralen chemischen Reinigungsmittels.

Der VDI-Wärmeatlas (10 Auflage, Springer Verlag 2006, ISBN-10 3-540-25504-4) offenbart auf Seite Od 23 in Paragraph 5.1.1 als generelle Vorgehensweise zunächst die Entfernung von organischen Ablagerungen mit einer Lauge, dann das Auswaschen mit Wasser gefolgt von einer Lockerung oder Lösung mit einer Säure, erneutes Spülen mit Wasser und anschließende Passivierung. In Beispiel 9 wird zudem angegeben, dass in einem bestimmten Fall die Behandlung von Siliciumdioxid- und Calciumphosphat-haltigen Ablagerungen mit einer Kombination von Natronlauge und einem Netzmittel bei 100 °C diese leichter löslich macht. Anschließend wird mit Salzsäure und Ammoniumbifluorid bei 70 °C behandelt, wobei das Ammoniumbifluorid eine geringe Menge Flusssäure erzeugt, die das Siliciumdioxid auflöst.

Die koreanische Patentanmeldung KR 2019 0004557 A befasst sich mit einem Verfahren zur Verbesserung der Säurebeständigkeit eines Rohrbündelwärmeaustauschers, in welchem Luft oder Wasser durch ein Heizgas indirekt erhitzt werden sollen. Dabei durchströmt die zu erwärmende Luft bzw. das zu erwärmende Wasser das Innere der Heizrohre, während das Heizgas diese von außen umfließt. Da als Heizgas ein Gas eingesetzt werden soll, das saure Bestandteile enthält, besteht die Gefahr, dass es durch die Abkühlung des Heizgases zu einem Niederschlag dieser sauren Bestandteile an der Außenseite der Heizrohre und infolgedessen zu Korrosion kommt. Nach der Lehre der KR 2019 0004557 wird eine derartige Korrosion durch eine spezielle Beschichtung der Rohraußenseite verhindert. Zu diesem Zweck werden die Heizrohre mit einer Glasur beschichtet und anschließend kalziniert. Als Glasur dient eine Mischung umfassend Siliciumdioxid, ein Nanometall linearer Struktur, verschiedene Metalloxide und Metallsalze sowie aus der Pyrolyse von Biomasse gewonnene Holzkohle, ein, insbesondere anorganisches, Pigment und Silikonöl. Zur Vorbereitung der Aufbringung der Glasur kann die Oberfläche der Heizrohre mit einer Ätzflüssigkeit enthaltend Schwefelsäure, Ascorbinsäure und ein Anionenaustauscherharz behandelt werden. Die Schrift befasst sich nicht mit der Aufkonzentrierung von Mineralsäuren unter deren Erhitzung. Vielmehr wird das säurehaltige Heizgas in dem beschriebenen Verfahren abgekühlt. Die Schrift befasst sich ebenfalls nicht mit der Reinigung des einzusetzenden Wärmeaustauschers, sondern nur damit, wie dieser auszugestalten ist, um möglichst wenig korrosionsanfällig zu sein.

Die japanische Patentanmeldung JP 2018 151132 A befasst sich mit einem Verfahren zur Reinigung eines Wärmeaustauschers, in welchem zuerst eine Reinigung mit einer ersten Reinigungsflüssigkeit erfolgt, woran sich eine Reinigung mit einer zweiten Reinigungsflüssigkeit anschließt. Das Verfahren stellt insbesondere auch eine Methode bereit, mit welcher der Reinigungsfortschritt kontrolliert werden kann und ist besonders geeignet für solche Wärmeaustauscher, die im Betrieb zur Bildung von Calcium- oder Silicium-haltigen Ablagerungen neigen, zum Beispiel Wassererhitzer oder Wärmeaustauscher zur Aufkonzentrierung von Reinigungswasser für Halbleiter. Als erste Reinigungsflüssigkeit eignet sich insbesondere eine wässrige Lösung enthaltend eine Carbonsäure, Sulfaminsäure, Methansulfonsäure und/oder deren Salze. Als zweite Reinigungsflüssigkeit werden in einer Ausführungsform wässrige Lösungen eines Hydrogendifluorids wie Ammonium-, Kalium- oder Natriumhydrogendifluorid beschrieben, während in einer anderen Ausführungsform der Einsatz alkalischer Lösungen wie Natrium- oder Kaliumhydroxid vorgesehen ist. Welche Ausführungsform vorzugsweise einzusetzen ist, hängt vom Material des Wärmeaustauschers ab. So wird der Einsatz der letztgenannten Ausführungsform unter Einsatz alkalischer Reinigungsflüssigkeiten für solche Wärmeaustauscher empfohlen, die aus Edelstahl oder Titan bestehen. Die Schrift befasst sich nicht mit Wärmeaustauschern, in denen Mineralsäuren aufkonzentriert werden sollen.

Die internationale Patentanmeldung WO 2011/032659 A1 betrifft ein Verfahren und eine Anlage zur Aufarbeitung, d. h. Auftrennung, Reinigung und Konzentrierung verbrauchter und verwässerter Schwefelsäure aus Nitrierprozessen ("Abfallsäure"), bei denen Salpetersäure in Gegenwart von Schwefelsäure als Nitriermedium eingesetzt wird. Das Verfahren ist insbesondere dadurch gekennzeichnet, dass man in einer ersten Stufe die vorgewärmte Abfallsäure in einer Strippkolonne in mindestens eine dampfförmige Phase, die Salpetersäure sowie gegebenenfalls Nitroorganika enthält, sowie eine vorkonzentrierte Schwefelsäure auftrennt, und man in nachgeschalteten Verfahrensstufen (i) die vorkonzentrierte Schwefelsäure einer weiteren Reinigung zur Abtrennung von Nitroorganika und einer Höherkonzentrierung zuführt und (ii) die aus der dampfförmigen Salpetersäurephase gewonnene Salpetersäure sowie die Nitroorganika, einschließlich der bei der weiteren Reinigung und Aufkonzentrierung der vorkonzentrierten Schwefelsäure erhaltenen Nitroorganika, aufarbeitet und wieder in das Nitrierverfahren zurückführt, wobei man in der ersten Stufe des Verfahrens zusätzlich zum Strippen der vorgewärmten Abfallsäure eine Aufkonzentrierung der im Strippdampf enthaltenen Salpetersäure durchführt, und wobei man die vom Kopf der Kolonne der ersten Stufe erhaltenen Salpetersäuredämpfe kondensiert und dabei eine Salpetersäure direkt in einer für eine Rückführung in das Nitrierverfahren geeigneten hochkonzentrierten Form gewinnt.

Der zuvor geschilderte Stand der Technik löst nicht die eingangs geschilderten Probleme. Es bestand daher ein Bedarf an weiteren Verbesserungen auf dem Gebiet der Reinigung von Vorrichtungen, die in der Aufkonzentrierung von Mineralsäuren eingesetzt werden. Insbesondere war es wünschenswert, ein Reinigungsverfahren zur Verfügung zu stellen, das einfach durchzuführen ist, ein Auseinandernehmen von Bauteilen der zu reinigenden Vorrichtung so weit wie möglich verzichtbar macht und den säurebeständigen Materialien möglichst keinen Schaden zufügt, weder durch mechanische noch chemische Belastungen.

Diesem Bedarf Rechnung tragend ist ein Gegenstand der vorliegenden Erfindung ein **Verfahren zur Vorbereitung einer Vorrichtung (oder einer Anlage umfassend mehrere Vorrichtungen) für deren Einsatz in einem (insbesondere kontinuierlich betriebenen) Prozess zur Aufkonzentrierung einer Mineralsäure durch Verdampfen von Wasser (also ein Verfahren zur Reinigung der Vorrichtung bzw. der Anlage oder eines Teils davon derart, dass die Vorrichtung bzw. die Anlage in einem solchen Prozess zur Aufkonzentrierung einer Mineralsäure eingesetzt werden kann),**
wobei die Vorrichtung (mindestens) eine gegen die Mineralsäure beständige Einrichtung umfasst,
wobei die Einrichtung Beschichtungen ausgewählt aus der Gruppe bestehend aus den Materialien
   Stahlemail, Siliciumcarbid, Glas (insbesondere Borosilikatglas), Tantal, Niob, perfluorierten Kunststoffen (wie insbesondere Polytetrafluorethylen und/oder Perfluoralkoxy-Polymere) und einem Verbund aus zwei oder mehr dieser Materialien
aufweist,
   oder
wobei die Einrichtung aus (mindestens) einem dieser Materialien gefertigt ist,
umfassend einen Schritt des Spülens der Einrichtung mit einer wässrigen Alkalimetallhydroxidlösung einer (auf die Gesamtmasse der Alkalimetallhydroxidlösung bezogenen) Massenkonzentration an Alkalimetallhydroxid im Bereich von 1 % bis 30 % bei einer Temperatur im Bereich von 40 °C bis 90 °C für einen Zeitraum von 2 Stunden bis 7 Tagen.

In der Terminologie der vorliegenden Erfindung bezeichnet eine säurebeständige *Einrichtung* einen Bestandteil der *Vorrichtung,* der mit der aufzukonzentrierenden Mineralsäure in direkten Kontakt tritt, also beispielsweise der Rohrinnen- oder Rohraußenraum eines Rohrbündelwärmeaustauschers. Der erfindungsgemäße Begriff der *Vorrichtung* umfasst auch den Fall, dass mehrere einzelne Apparate, die sich jeweils als *Vorrichtung* im hier verwendeten Sinne qualifizieren, zu einem Verbund mehrerer *Vorrichtungen,* auch als *Anlage* bezeichnet, zusammengeschlossen sind. Dabei kann eine Anlage neben zu reinigenden Vorrichtungen selbstverständlich noch weitere Apparate umfassen.

Es folgt zunächst eine Kurzzusammenfassung verschiedener möglicher **Ausführungsformen der Erfindung:**
In einer **ersten Ausführungsform** der Erfindung, die mit allen anderen Ausführungsformen kombiniert werden kann, wird die Alkalimetallhydroxidlösung ausgewählt aus der Gruppe bestehend aus Natronlauge, Kalilauge und Mischungen derselben.

In einer **zweiten Ausführungsform** der Erfindung, die mit allen anderen Ausführungsformen kombiniert werden kann, liegt die Massenkonzentration an Alkalimetallhydroxid in der Alkalimetallhydroxidlösung im Bereich von 3 % bis 20 %.

In einer **dritten Ausführungsform** der Erfindung, die eine besondere Ausgestaltung der zweiten Ausführungsform ist, liegt die Massenkonzentration an Alkalimetallhydroxid in der Alkalimetallhydroxidlösung im Bereich von 5 % bis 15 %.

In einer **vierten Ausführungsform** der Erfindung, die mit allen anderen Ausführungsformen kombiniert werden kann, wird das Spülen bei einer Temperatur im Bereich von 50 °C bis 80 °C durchgeführt.

In einer **fünften Ausführungsform** der Erfindung, die eine besondere Ausgestaltung der vierten Ausführungsform ist, wird das Spülen bei einer Temperatur im Bereich von 55 °C bis 70 °C durchgeführt.

In einer **sechsten Ausführungsform** der Erfindung, die mit allen anderen Ausführungsformen kombiniert werden kann, wird das Spülen für einen Zeitraum von 8 Stunden bis 30 Stunden durchgeführt.

In einer **siebten Ausführungsform** der Erfindung, die eine besondere Ausgestaltung der sechsten Ausführungsform ist, wird das Spülen für einen Zeitraum von 12 Stunden bis 24 Stunden durchgeführt.

In einer **achten Ausführungsform** der Erfindung, die mit allen anderen Ausführungsformen kombiniert werden kann, umfasst die Vorrichtung einen Wärmeaustauscher zur Erwärmung der aufzukonzentrierenden Mineralsäure, eine Destillationsapparatur zur Entfernung organischer Verunreinigungen aus der aufzukonzentrierenden Mineralsäure, einen Verdampfungsapparat (wobei mehrere Verdampfungsapparate in Reihe geschaltet werden können), einen Wärmeaustauscher zur Abkühlung der aufkonzentrierten Mineralsäure und/oder einen Behälter zur Aufnahme der aufkonzentrierten Mineralsäure.

In einer **neunten Ausführungsform** der Erfindung, die eine besondere Ausgestaltung der achten Ausführungsform ist, ist die Vorrichtung ein Wärmeaustauscher, der zur Erwärmung der aufzukonzentrierenden Mineralsäure und/oder zur Abkühlung der aufkonzentrierten Mineralsäure ausgelegt ist.

In einer **zehnten Ausführungsform** der Erfindung, die eine weitere besondere Ausgestaltung der achten Ausführungsform ist, umfasst die Vorrichtung einen Wärmeaustauscher zur Abkühlung der aufkonzentrierten Mineralsäure und einen diesem nachgeschalteter Behälter zur Aufnahme der aufkonzentrierten Mineralsäure.

In einer **elften Ausführungsform** der Erfindung, die mit allen anderen Ausführungsformen kombiniert werden kann, ist die Mineralsäure ausgewählt aus der Gruppe bestehend aus Schwefelsäure, Salpetersäure und Mischungen derselben.

In einer **zwölften Ausführungsform** der Erfindung, die eine besondere Ausgestaltung der elften Ausführungsform ist, wird der Prozess zur Aufkonzentrierung der Mineralsäure durch Verdampfen von Wasser dazu eingesetzt, Schwefelsäure, die als Reaktionsmedium in einer Nitrierung einer aromatischen Verbindung mit Salpetersäure zur Gewinnung einer aromatischen Nitroverbindung eingesetzt und durch die Nitrierung verdünnt wird, wieder auf ihre ursprünglich in der Nitrierung eingesetzte Konzentration aufzukonzentrieren. Mit anderen Worten ist der Prozess zur Aufkonzentrierung der Mineralsäure durch Verdampfen von Wasser in dieser zwölften Ausführungsform Bestandteil eines Verfahrens zur Herstellung einer aromatischen Nitroverbindung durch Nitrierung einer aromatischen Verbindung mit Salpetersäure in Gegenwart von Schwefelsäure, wobei der Prozess zur Aufkonzentrierung der Mineralsäure dazu eingesetzt wird, die im Zuge der Nitrierung verdünnte Schwefelsäure wieder auf ihre ursprünglich in der Nitrierung eingesetzte Konzentration aufzukonzentrieren.

In einer **dreizehnten Ausführungsform** der Erfindung, die eine besondere Ausgestaltung der zwölften Ausführungsform ist, ist die aromatische Nitroverbindung Nitrobenzol oder Dinitrotoluol, bevorzugt Dinitrotoluol.

In einer **vierzehnten Ausführungsform** der Erfindung, die eine weitere besondere Ausgestaltung der elften Ausführungsform ist, wird der Prozess zur Aufkonzentrierung der Mineralsäure durch Verdampfen von Wasser dazu eingesetzt, Schwefelsäure, die als Trockenmittel zur Trocknung eines Gases eingesetzt und durch das Trocknen verdünnt wird, wieder auf ihre ursprünglich in der Trocknung eingesetzte Konzentration aufzukonzentrieren. Mit anderen Worten ist der Prozess zur Aufkonzentrierung der Mineralsäure durch Verdampfen von Wasser in dieser vierzehnten Ausführungsform Bestandteil eines Verfahrens zur Trocknung eines Gases, bei welchem Schwefelsäure als Trockenmittel eingesetzt wird, wobei der Prozess zur Aufkonzentrierung der Mineralsäure dazu eingesetzt wird, die im Zuge der Trocknung verdünnte Schwefelsäure wieder auf ihre ursprünglich in der Trocknung eingesetzte Konzentration aufzukonzentrieren.

In einer **fünfzehnten Ausführungsform** der Erfindung, die eine besondere Ausgestaltung der vierzehnten Ausführungsform ist, ist das Gas Chlor.

In einer **sechzehnten Ausführungsform** der Erfindung, die mit allen anderen Ausführungsformen kombiniert werden kann, weist die Einrichtung Beschichtungen auf, die perfluorierte Kunststoffe (wie insbesondere Polytetrafluorethylen und/oder Perfluoralkoxy-Polymere) umfassen bzw. aus diesen bestehen, oder ist die Einrichtung zumindest teilweise aus perfluorierten Kunststoffen (wie insbesondere Polytetrafluorethylen und/oder Perfluoralkoxy-Polymere) gefertigt, wobei die perfluorierten Kunststoffe (in beiden Fällen) mit Inerststoffen (insbesondere Glaskugeln, Glasperlen sowie Aluminat oder Silikat enthaltende Tonerden) mechanisch stabilisiert sind.

In einer **siebzehnten Ausführungsform** der Erfindung, die mit allen anderen Ausführungsformen kombiniert werden kann, sofern diese nicht auf den Einsatz perfluorierter Kunststoffe als gegen die Mineralsäure beständige Materialien beschränkt sind, weist die Einrichtung Beschichtungen auf, die Stahlemail, Siliciumcarbid, Glas (insbesondere Borosilikatglas), Niob und/oder Tantal umfassen bzw. aus diesen bestehen, oder ist die Einrichtung zumindest teilweise aus Stahlemail, Siliciumcarbid, Glas (insbesondere Borosilikatglas), Niob und/oder Tantal gefertigt.

In einer **achtzehnten Ausführungsform** der Erfindung, die mit allen anderen Ausführungsformen kombiniert werden kann, wird die Einrichtung zum Zwecke der Durchführung des Spülens nicht auseinandergebaut.

In einer **neunzehnten Ausführungsform** der Erfindung, die eine besondere Ausgestaltung der achtzehnten Ausführungsform ist, wird die (ganze) Vorrichtung zum Zwecke der Durchführung des Spülens der Einrichtung nicht auseinandergebaut.

Die zuvor kurz geschilderten Ausführungsformen und weitere mögliche Ausgestaltungen der Erfindung werden im Folgenden näher erläutert. Verschiedene Ausführungsformen sind, sofern sich aus dem Kontext für den Fachmann nicht eindeutig das Gegenteil ergibt, beliebig miteinander kombinierbar.

Als Alkalimetallhydroxidlösung wird vorzugsweise Natronlauge und/oder Kalilauge eingesetzt. Unabhängig von der Art der eingesetzten Alkalimetallhydroxidlösung liegt deren Massenkonzentration an Alkalimetallhydroxid bevorzugt im Bereich von 3 % bis 20 % und besonders bevorzugt im Bereich von 5 % bis 15 %.

Das Spülen mit der Alkalimetallhydroxidlösung wird vorzugsweise bei Temperaturen im Bereich von 50 °C bis 80 °C, besonders bevorzugt im Bereich von 55 °C bis 70 °C, über einen Zeitraum von bevorzugt 8 Stunden bis 30 Stunden, besonders bevorzugt von 12 Stunden bis 24 Stunden, durchgeführt.

Das erfindungsgemäße Verfahren ist prinzipiell auf alle Vorrichtungen, die in der Aufkonzentrierung von Mineralsäuren Anwendung finden, anwendbar. So kann es sich etwa um einen Wärmeaustauscher zur Erwärmung der aufzukonzentrierenden Mineralsäure, eine Destillationsapparatur zur Entfernung organischer Verunreinigungen aus der aufzukonzentrierenden Mineralsäure, einen Verdampfungsapparat (wobei mehrere Verdampfungsapparate in Reihe geschaltet werden können), einen Wärmeaustauscher zur Abkühlung der aufkonzentrierten Mineralsäure und/oder einen Behälter zur Aufnahme der aufkonzentrierten Mineralsäure handeln. Dabei ist hier und im Folgenden von Formulierungen wie *"ein Wärmeaustauscher", "eine Destillationsapparatur"* und dergleichen selbstredend die Möglichkeit umfasst, dass mehrere solcher Apparate vorhanden sind und unter Einsatz des erfindungsgemäßen Verfahrens gereinigt werden.

In bevorzugter Ausgestaltung der Erfindung wird das erfindungsgemäße Reinigungsverfahren für die Reinigung eines Wärmeaustauschers eingesetzt, der zur Erwärmung der aufzukonzentrierenden Mineralsäure und/oder zur Abkühlung der aufkonzentrierten Mineralsäure ausgelegt ist. Das erfindungsgemäße Reinigungsverfahren eignet sich auch zur Reinigung einer Anlage, die einen Wärmeaustauscher zur Abkühlung der aufkonzentrierten Mineralsäure und einen diesem nachgeschalteter Behälter zur Aufnahme der aufkonzentrierten Mineralsäure umfasst.

Prinzipiell ist das erfindungsgemäße Reinigungsverfahren auf Vorrichtungen für die Aufkonzentrierung beliebiger Mineralsäuren anwendbar. Besonders bevorzugt sind Schwefelsäure, Salpetersäure und Mischungen derselben. Schwefel- und Salpetersäure finden Anwendung in der Herstellung einer aromatischer Nitroverbindungen durch Nitrierung von aromatischen Verbindungen. Die Salpetersäure dient als Quelle für die Nitrogruppe, während die Schwefelsäure als Katalysator und Verdünnungsmittel fungiert. In adiabatisch betriebenen Verfahren nimmt die Schwefelsäure außerdem die (beträchtliche) Reaktionswärme auf.

Vorzugsweise wird in Aromaten-Nitrierverfahren der zu nitrierende Aromat im Überschuss eingesetzt, sodass die Salpetersäure (bis auf möglicherweise Spurenanteile) vollständig verbraucht wird. Die Schwefelsäure wird jedoch lediglich verdünnt und soll, bis auf gegebenenfalls Anteile, die zur Entfernung von Verunreinigungen ausgeschleust werden, nach Aufkonzentrierung auf die ursprünglich in der Nitrierung eingesetzte Konzentration wieder in das Nitrierverfahren zurückgeführt werden.

Als zu nitrierende Aromaten eignen sich insbesondere Benzol und Toluol. Benzol wird dabei zu Nitrobenzol mononitriert und Toluol zu Dinitrotoluol dinitriert. Bevorzugt ist die Anwendung des erfindungsgemäßen Verfahrens in der Herstellung von Dinitrotoluol.

Ein anderes Anwendungsgebiet für das erfindungsgemäße Verfahren sind Prozesse, in denen Schwefelsäure als Trockenmittel zur Gastrocknung eingesetzt und im Zuge dessen verdünnt wird. In solchen Prozessen muss die Schwefelsäure nach Aufnahme einer gewissen Menge Wassers wieder auf die ursprünglich in der Trocknung eingesetzte Konzentration aufkonzentriert werden. Als Beispiel für eine solche Anwendung sei die Trocknung feuchten Chlorgases genannt.

Werden als säurebeständige Materialien perfluorierte Kunststoffe eingesetzt, so sind Polytetrafluorethylen und/oder Perfluoralkoxy-Polymere bevorzugt. Insbesondere wenn solche Kunststoffe nicht lediglich als Beschichtungen eingesetzt werden, sondern die Einrichtungen, die mit der Mineralsäure in Berührung kommen können, aus solchen Kunststoffen gefertigt sind, ist es bevorzugt, die perfluorierten Kunststoffe mit Inerststoffen mechanisch zu stabilisieren. Als Inertstoffe eignen sich dabei bevorzugt Glaskugeln oder -perlen oder auch auf Aluminat oder Silikat enthaltende Tonerden.

Andere geeignete säurebeständige Materialien sind Stahlemail, Siliciumcarbid, Glas (insbesondere Borosilikatglas), Niob und/oder Tantal. Diese können als Beschichtungen oder als Werkstoff, aus dem die mit der Mineralsäure in Kontakt tretenden Einrichtungen gefertigt sind, eingesetzt werden.

Wird das säurebeständige Material lediglich als Beschichtung eingesetzt, so können als Werkstoffe für die Einrichtungen, die mit der Mineralsäure während des Betriebs der Vorrichtung in Kontakt treten und auf denen die Beschichtungen aufgebracht werden, alle dem Fachmann geläufigen Materialien in Frage, insbesondere Edelstahl oder Schwarzstahl.

Das erfindungsgemäße Verfahren bietet den großen Vorteil, dass es ohne ein Auseinanderbauen der zu spülenden Einrichtung durchgeführt werden kann. Es kann jedoch erforderlich sein, Rohrleitungen, die einzelne Einrichtungen miteinander oder die Einrichtung mit anderen Bestandteilen der Vorrichtung oder weiteren Apparaten verbinden, auseinanderzunehmen. Die Alkalimetallhydroxidlösung kann über ohnehin vorhandenen Eintrittsöffnungen (etwa die Eintrittsöffnung für die Zufuhr der aufzukonzentrierenden Mineralsäure im regulären Betrieb der Vorrichtung) eingeführt und über ohnehin vorhandenen Austrittsöffnungen (etwa für die Abfuhr der aufkonzentrierten Mineralsäure im regulären Betrieb der Vorrichtung) abgeführt werden. Es ist jedoch selbstverständlich auch möglich, eigens zum Zweck des Spülens vorgesehene, verschließbare (und während des regulären Betriebs der Vorrichtung verschlossene) Eintritts- und Austrittsöffnungen an geeigneten Stellen der Vorrichtung anzubringen, sodass diese zum Zwecke des Spülens lediglich geöffnet werden müssen. In einem solchen Fall ist es möglich, dass die Vorrichtung nicht einmal teilweise auseinandergebaut werden muss (also auch alle im Regelbetrieb vorhandenen Rohrleitungen montiert bleiben können).

Das erfindungsgemäße Verfahren wird nachstehend noch anhand von Beispielen näher ausgeführt.

### Beispiele:

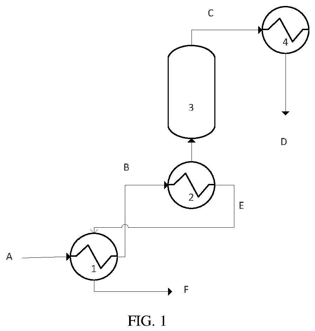

Eine Anlage zur Aufkonzentrierung von Abfallsäure aus einem Nitrierprozess von Toluol zu Dinitrotoluol (DNT) ist in vereinfachter Darstellung in FIG. 1 gezeigt. Sie besteht aus mehreren Rekuperatoren (1), mehreren Verdampfern (2), die jeder mit Kolonnen (3) und Kondensatoren (4) verbunden sind (gezeigt ist jeweils nur einer dieser Apparate). Der Zulauf der Abfallsäure ist mit A bezeichnet, die aufkonzentrierte Säure mit F und das Prozesskondensat mit D. Es wurde eine starke Verschmutzung der Wärmeaustauscher (1) festgestellt, so dass die heiße, mit E bezeichnete, konzentrierte Säure aus dem Verdampfer (2) nicht mehr frei, wie für den Betrieb erforderlich, durch die Wärmeaustauscher (1) ablaufen konnte. Zudem wurde beobachtet, dass der Wärmedurchgang durch die Wärmeaustauscher (1) erheblich reduziert war, was sich durch eine verringerte Temperatur des Stroms B, eine erhöhte Temperatur des Stroms F und einen erhöhten Energieverbrauch der Verdampfer (2) bemerkbar machte.

Die Wärmeaustauscher (1) wurde zunächst mit Wasser und anschließend mit 10%iger Natronlauge gespült. Hierzu wurden die Teile der beiden Rohrleitungen B und E, die jeweils mit den Stutzen des letzten der Wärmeaustauscher (1) verbunden sind, demontiert und die beiden Stutzen durch einen Schlauch verbunden. Während dieser Tätigkeit wurden die Stutzen inspiziert und große Mengen an festen Ablagerungen gefunden. Anschließend wurde die auf 65 °C erwärmte Natronlauge aus einer Vorlage für 24 Stunden durch den am Wärmeaustauscher (1) in der Leitung F befindlichen Spülstutzen gepumpt und an einem in der Leitung A befindlichen Spülstutzen entnommen und in die Vorlage zurückgeführt. Nach einer erneuten kurzen Spülung mit Wasser wurde der Schlauch demontiert und die Stutzen erneut inspiziert. Es wurde keinerlei Verschmutzung mehr beobachtet, die Reinigung war demzufolge vollständig. Die entfernten Rohrleitungen wurden wieder montiert und die Anlage wieder in Betrieb genommen. Dabei wurde festgestellt, dass die Säure wieder frei ablaufen konnte und der Wärmedurchgang durch die Wärmeaustauscher (1) dem Auslegungswert entspricht.

## Patentansprüche

1. Verfahren zur Vorbereitung einer Vorrichtung für deren Einsatz in einem Prozess zur Aufkonzentrierung einer Mineralsäure durch Verdampfen von Wasser, wobei die Vorrichtung eine gegen die Mineralsäure beständige Einrichtung umfasst,
wobei die Einrichtung Beschichtungen ausgewählt aus der Gruppe bestehend aus den Materialien
Stahlemail, Siliciumcarbid, Glas, Tantal, Niob, perfluorierten Kunststoffen und einem Verbund aus zwei oder mehr dieser Materialien
aufweist,
oder
wobei die Einrichtung aus einem dieser Materialien gefertigt ist,
umfassend einen Schritt des Spülens der Einrichtung mit einer wässrigen Alkalimetallhydroxidlösung einer Massenkonzentration an Alkalimetallhydroxid im Bereich von 1 % bis 30 % bei einer Temperatur im Bereich von 40 °C bis 90 °C für einen Zeitraum von 2 Stunden bis 7 Tagen.

2. Verfahren nach Anspruch 1, bei welchem die Alkalimetallhydroxidlösung ausgewählt ist aus der Gruppe bestehend aus Natronlauge, Kalilauge und Mischungen derselben.

3. Verfahren nach einem der vorstehenden Ansprüche, bei welchem das Spülen für einen Zeitraum von 8 Stunden bis 30 Stunden durchgeführt wird.

4. Verfahren nach einem der vorstehenden Ansprüche, bei welchem die Vorrichtung einen Wärmeaustauscher zur Erwärmung der aufzukonzentrierenden Mineralsäure, eine Destillationsapparatur zur Entfernung organischer Verunreinigungen aus der aufzukonzentrierenden Mineralsäure, einen Verdampfungsapparat, einen Wärmeaustauscher zur Abkühlung der aufkonzentrierten Mineralsäure und/oder einen Behälter zur Aufnahme der aufkonzentrierten Mineralsäure umfasst.

5. Verfahren nach Anspruch 4, bei welchem die Vorrichtung ein Wärmeaustauscher ist, der zur Erwärmung der aufzukonzentrierenden Mineralsäure und/oder zur Abkühlung der aufkonzentrierten Mineralsäure ausgelegt ist.

6. Verfahren nach Anspruch 4, bei welchem die Vorrichtung einen Wärmeaustauscher zur Abkühlung der aufkonzentrierten Mineralsäure und einen diesem nachgeschalteter Behälter zur Aufnahme der aufkonzentrierten Mineralsäure umfasst.

7. Verfahren nach einem der vorstehenden Ansprüche, bei welchem die Mineralsäure ausgewählt ist aus der Gruppe bestehend aus Schwefelsäure, Salpetersäure und Mischungen derselben.

8. Verfahren nach Anspruch 7, bei welchem der Prozess zur Aufkonzentrierung der Mineralsäure durch Verdampfen von Wasser dazu eingesetzt wird, Schwefelsäure, die als Reaktionsmedium in einer Nitrierung einer aromatischen Verbindung mit Salpetersäure zur Gewinnung einer aromatischen Nitroverbindung eingesetzt und durch die Nitrierung verdünnt wird, wieder auf ihre ursprünglich in der Nitrierung eingesetzte Konzentration aufzukonzentrieren.

9. Verfahren nach Anspruch 8, bei welchem die aromatische Nitroverbindung Nitrobenzol oder Dinitrotoluol ist.

10. Verfahren nach Anspruch 7, bei welchem der Prozess zur Aufkonzentrierung der Mineralsäure durch Verdampfen von Wasser dazu eingesetzt wird, Schwefelsäure, die als Trockenmittel zur Trocknung eines Gases eingesetzt und durch das Trocknen verdünnt wird, wieder auf ihre ursprünglich in der Trocknung eingesetzte Konzentration aufzukonzentrieren.

11. Verfahren nach Anspruch 10, bei welchem das Gas Chlor ist.

12. Verfahren nach einem der vorstehenden Ansprüche, bei welchem die Einrichtung Beschichtungen aufweist, die perfluorierte Kunststoffe umfassen bzw. aus diesen bestehen, oder bei welchem die Einrichtung zumindest teilweise aus perfluorierten Kunststoffen gefertigt ist, wobei die perfluorierten Kunststoffe mit Inerststoffen mechanisch stabilisiert sind.

13. Verfahren nach einem der Ansprüche 1 bis 11, bei welchem die Einrichtung Beschichtungen aufweist, die Stahlemail, Siliciumcarbid, Glas, Niob und/oder Tantal umfassen bzw. aus diesen bestehen, oder bei welchem die Einrichtung zumindest teilweise aus Stahlemail, Siliciumcarbid, Glas, Niob und/oder Tantal gefertigt ist.

14. Verfahren nach einem der vorstehenden Ansprüche, bei welchem die Einrichtung zum Zwecke der Durchführung des Spülens nicht auseinandergebaut wird.

15. Verfahren nach Anspruch 14, bei welchem die Vorrichtung zum Zwecke der Durchführung des Spülens der Einrichtung nicht auseinandergebaut wird.

## Claims

1. Method for preparing an apparatus for use in a process for concentrating a mineral acid by evaporation of water, wherein the apparatus comprises a device which is resistant to the mineral acid,
wherein the device has coatings selected from the group consisting of the materials
steel enamel, silicon carbide, glass, tantalum, niobium, perfluorinated polymers and a composite of two or more of these materials,
or
wherein the device is made of one of these materials,
comprising a step of flushing of the device with an aqueous alkali metal hydroxide solution having a concentration by mass of alkali metal hydroxide in the range from 1% to 30% at a temperature in the range from 40°C to 90°C for a time of from 2 hours to 7 days.

2. Method according to Claim 1, wherein the alkali metal hydroxide solution is selected from the group consisting of sodium hydroxide solution, potassium hydroxide solution and mixtures thereof.

3. Method according to either of the preceding claims, wherein flushing is carried out for a time of from 8 hours to 30 hours.

4. Method according to any of the preceding claims, wherein the apparatus comprises a heat exchanger for heating the mineral acid to be concentrated, a distillation apparatus for removing organic contaminants from the mineral acid to be concentrated, a vaporization apparatus, a heat exchanger for cooling the mineral acid which has been concentrated and/or a vessel for accommodating the mineral acid which has been concentrated.

5. Method according to Claim 4, wherein the apparatus is a heat exchanger which is designed for heating the mineral acid to be concentrated and/or for cooling the mineral acid which has been concentrated.

6. Method according to Claim 4, wherein the apparatus comprises a heat exchanger for cooling the mineral acid which has been concentrated and a vessel downstream thereof for accommodating the mineral acid which has been concentrated.

7. Method according to any of the preceding claims, wherein the mineral acid is selected from the group consisting of sulfuric acid, nitric acid and mixtures thereof.

8. Method according to Claim 7, wherein the process of concentrating the mineral acid by evaporation of water is used to concentrate sulfuric acid, which is used as reaction medium in a nitration of an aromatic compound with nitric acid to obtain an aromatic nitro compound and is diluted by the nitration, back to its concentration originally used in the nitration.

9. Method according to Claim 8, wherein the aromatic nitro compound is nitrobenzene or dinitrotoluene.

10. Method according to Claim 7, wherein the process of concentrating the mineral acid by evaporation of water is used for concentrating sulfuric acid, which is used as desiccant for drying a gas and is diluted by the drying, back to its concentration originally used in the drying.

11. Method according to Claim 10, wherein the gas is chlorine.

12. Method according to any of the preceding claims, wherein the device has coatings which comprise or consist of perfluorinated polymers or wherein the device is made at least partly of perfluorinated polymers, where the perfluorinated polymers are mechanically stabilized with inert materials.

13. Method according to any of Claims 1 to 11, wherein the device has coatings which comprise or consist of steel enamel, silicon carbide, glass, niobium and/or tantalum or wherein the device is made at least partly of steel enamel, silicon carbide, glass, niobium and/or tantalum.

14. Method according to any of the preceding claims, wherein the device is not disassembled for the purpose of carrying out the flushing.

15. Method according to Claim 14, wherein the apparatus is not disassembled for the purpose of carrying out the flushing of the device.

## Revendications

1. Procédé de préparation d'un dispositif en vue de son utilisation dans un procédé de concentration d'un acide minéral par évaporation d'eau, le dispositif comprenant une équipement résistant à l'acide minéral,
l'équipement présentant des revêtements choisis dans le groupe constitué par les matériaux
acier émaillé, carbure de silicium, verre, tantale, niobium, matériaux synthétiques perfluorés et un composite de deux de ces matériaux ou plus,
ou
l'équipement étant fabriqué à partir d'un de ces matériaux,
comprenant une étape de rinçage de l'équipement à l'aide d'une solution aqueuse d'hydroxyde de métal alcalin d'une concentration massique en hydroxyde de métal alcalin dans la plage de 1 % à 30 % à une température dans la plage de 40 °C à 90 °C pendant une période de 2 heures à 7 jours.

2. Procédé selon la revendication 1, dans lequel la solution d'hydroxyde de métal alcalin est choisie dans le groupe constitué par la lessive de soude caustique, la lessive potassique et leurs mélanges.

3. Procédé selon l'une des revendications précédentes, dans lequel le rinçage est réalisé pendant une période de 8 heures à 30 heures.

4. Procédé selon l'une des revendications précédentes, dans lequel le dispositif comprend un échangeur de chaleur destiné à réchauffer l'acide minéral à concentrer, un appareil de distillation destiné à éliminer les impuretés organiques de l'acide minéral à concentrer, un appareil d'évaporation, un échangeur de chaleur destiné à refroidir l'acide minéral concentré et/ou un récipient destiné à recevoir l'acide minéral concentré.

5. Procédé selon la revendication 4, dans lequel le dispositif est un échangeur de chaleur qui est conçu pour réchauffer l'acide minéral à concentrer et/ou pour refroidir l'acide minéral concentré.

6. Procédé selon la revendication 4, dans lequel le dispositif comprend un échangeur de chaleur destiné à refroidir l'acide minéral concentré et un récipient disposé en aval de celui-ci destiné à recevoir l'acide minéral concentré.

7. Procédé selon l'une des revendications précédentes, dans lequel l'acide minéral est choisi dans le groupe constitué par l'acide sulfurique, l'acide nitrique et leurs mélanges.

8. Procédé selon la revendication 7, dans lequel le procédé de concentration de l'acide minéral par évaporation d'eau est utilisé pour concentrer de l'acide sulfurique, qui est utilisé comme milieu de réaction dans une nitration d'un composé aromatique avec de l'acide nitrique afin d'obtenir un composé nitro aromatique et qui est dilué par la nitration, de nouveau à sa concentration initiale utilisée dans la nitration.

9. Procédé selon la revendication 8, dans lequel le composé nitro aromatique est le nitrobenzène ou le dinitrotoluène.

10. Procédé selon la revendication 7, dans lequel le procédé de concentration de l'acide minéral par évaporation d'eau est utilisé pour concentrer de l'acide sulfurique, qui est utilisé comme dessiccateur en vue du séchage d'un gaz et qui est dilué par le séchage, de nouveau à sa concentration initiale utilisée dans le séchage.

11. Procédé selon la revendication 10, dans lequel le gaz est du chlore.

12. Procédé selon l'une des revendications précédentes, dans lequel l'équipement présente des revêtements qui comprennent des matériaux synthétiques perfluorés ou, selon le cas, qui sont constitués par ceux-ci ou dans lequel l'équipement est fabriqué au moins partiellement à partir de matériaux synthétiques perfluorés, les matériaux synthétiques perfluorés étant stabilisés mécaniquement avec des substances inertes.

13. Procédé selon l'une des revendications 1 à 11, dans lequel l'équipement présente des revêtements qui comprennent de l'acier émaillé, du carbure de silicium, du verre, du niobium et/ou du tantale ou, selon le cas, qui sont constitués par ceux-ci ou dans lequel l'équipement est fabriqué au moins partiellement à partir d'acier émaillé, de carbure de silicium, de verre, de niobium et/ou de tantale.

14. Procédé selon l'une des revendications précédentes, dans lequel l'équipement n'est pas démonté en vue de la réalisation du rinçage.

15. Procédé selon la revendication 14, dans lequel le dispositif n'est pas démonté en vue de la réalisation du rinçage.
